# EUROPÄISCHE PATENTANMELDUNG

(11) **EP 4 169 550 A1**
(43) Veröffentlichungstag der Anmeldung: **26.04.2023**
(21) Anmeldenummer: 22202306.1
(22) Anmeldetag: 18.10.2022
(51) Int. Cl.: A61M 5/145

(54) **INNERER AUFBAU EINER MEDIZINISCHEN FLUIDPUMPE**

(30) Priorität: 19.10.2021 DE 102021127062
(71) Anmelder: B. Braun Melsungen AG, 34212 Melsungen (DE)
(72) Erfinder: SCHWARZ, Jan, 34212 Melsungen (DE); ERLEN, Christoph, 34132 Kassel (DE); GERLACH, Hans-Josef, 34431 Marsberg (DE); KAUBA, Michael, 34277 Fuldabrück (DE); SCHWALM, Matthias, 34613 Schwalmstadt (DE); SCHÜTZ, Joachim, 36041 Fulda (DE); SCHÖFFEL, Gerhard, 89134 Blaustein (DE); WOLF, Daniel, 89346 Bibertal (DE)
(74) Vertreter: Winter, Brandl - Partnerschaft mbB

(57) **Zusammenfassung**

Die Offenbarung betrifft eine medizinische Fluidpumpe (2), insbesondere in Form einer Spritzen- oder Infusionspumpe (26; 54), mit einem Gehäuse (4), einer an einer Gehäusevorderseite angelenkten Frontklappe, einem schwenkbar an sich gegenüberliegenden ersten und zweiten Seitenflächen (44; 46) anscharnierten Tragegriff (18), der aus einer Lagerungsposition in eine Trageposition schwenkbar ist, in welcher er sich oberhalb des Gehäuses (4) anordnet, und einer Anzahl von elektrischen und mechanischen Bauteilen innerhalb des Gehäuses (4), die für sämtliche vorgesehenen Funktionen der medizinischen Fluidpumpe (2) erforderlich sind. Eine Auswahl an Bauteilen aus den elektrischen und mechanischen Bauteilen innerhalb des Gehäuses (4) sind als Tarierelemente vorgesehen, die innerhalb des Gehäuses (4) so platziert sind, dass sich ein Schwerpunkt der medizinischen Fluidpumpe (2) im Wesentlichen unterhalb des in Trageposition befindlichen Tragegriffs (18) befindet.

## Beschreibung

### Technisches Gebiet

Die vorliegende Offenbarung betrifft eine medizinische Fluidpumpe und deren inneren Aufbau.

### Hintergrund der Offenbarung

In der Medizin werden verbreitet Fluidpumpen, insbesondere Spritzenpumpen und Schlauchpumpen zur Versorgung eines Patienten mit einer definierten Dosis an Medikamenten eingesetzt. Da es sich bei solchen medizinischen Fluidpumpen um kostspielige medizinische Ausrüstung handelt, ist im Regelfall nicht jeder Patientenplatz mit einer solchen medizinischen Fluidpumpe ausgestattet, sondern es wird im Bedarfsfall die medizinische Fluidpumpe an dem Patientenplatz installiert. Aus diesem Grund handelt es sich bei solchen medizinischen Fluidpumpen häufig um mobile bzw. teilmobile Vorrichtungen, die von einem Behandler zu dem betreffenden Patienten bzw. Patientenplatz verbracht und dort installiert werden. Dies hat weiterhin den Vorteil, dass bei einem Verlegen des Patienten auf eine andere Station oder in einen Operationssaal der Patient nicht von der medizinischen Fluidpumpe getrennt werden muss und somit eine gleichmäßige, unterbrechungsfreie Versorgung des Patienten mit der definierten Dosis an Medikamenten sichergestellt ist.

Um eine einfache Handhabung und einen einfachen Transport für den Behandler zu gewährleisten, kann die medizinische Fluidpumpe mit einem Tragegriff versehen sein.

Nun hat sich gezeigt, dass es bei dem Transport der medizinischen Fluidpumpe an dem Tragegriff zu einem Verkippen des Gehäuses der medizinischen Fluidpumpe aus einer waagerechten Position heraus kommen kann. Dies ist gerade dann problematisch, wenn der Patient weiter von der medizinischen Fluidpumpe versorgt wird und es durch das Verkippen zu einer Beeinträchtigung der ordnungsgemäßen Funktion der medizinischen Fluidpumpe kommen kann.

Um dieses Problem zu lösen, wurden verschiedenste Lösungsansätze in Betracht gezogen. Der Tragegriff wurde in einer Trageposition relativ zu dem Gehäuse verriegelt, sodass das Verkippen des Gehäuses relativ zu dem Tragegriff in der Trageposition verhindert werden konnte. Dies hatte jedoch den Nachteil, dass weitere bewegliche Bauteile in Form der Verriegelung an der medizinischen Fluidpumpe vorgesehen werden mussten, die einerseits schwer zu reinigen sind und damit eine Brutstelle für Keime und Bakterien darstellen können und andererseits ein weiteres Bauteil darstellen, das die medizinische Fluidpumpe verteuert und eine weitere Schadensquelle an der medizinischen Fluidpumpe darstellt.

In einem weiteren Ansatz wurden Anlenkpunkte des Tragegriffs beweglich ausgeführt, um die Trageposition variabel zu gestalten, sodass der Tragegriff in der Trageposition über einen Schwerpunkt der Fluidpumpe bewegt werden konnte. Auch hier zeigte sich jedoch, dass die weiteren beweglichen Bauteile in Form eines verschiebbaren Lagers die medizinische Fluidpumpe verteuerten, eine weitere Schadensquelle darstellen und eine Handhabung der medizinischen Fluidpumpe deutlich erschweren.

Ein dritter Ansatz stellte ein Vorsehen von metallischen, insbesondere bleiernden Ausgleichsgewichten dar, die in dem Gehäuse der medizinischen Fluidpumpe so angebracht wurden, dass ein Schwerpunkt der medizinischen Fluidpumpe unterhalb des Tragegriffs in der Trageposition angeordnet ist. Das Einbringen von Ausgleichsgewichten führte jedoch dazu, dass sich das Gesamtgewicht der medizinischen Fluidpumpe erhöhte und dadurch die Handhabung für Behandler erschwert wurde.

### Zusammenfassung der Offenbarung

Es sind Aufgaben und Ziele der Offenbarung, die Nachteile aus dem Stand der Technik zu beheben oder wenigsten zu mindern und insbesondere eine medizinische Fluidpumpe mit einem Tragegriff bereitzustellen, wobei ein Verkippen eines Gehäuses gegen den Tragegriff in einer Trageposition verhindert ist. Insbesondere soll erreicht werden, dass in einer Trageposition das Gehäuse der Fluidpumpe im Wesentlichen horizontal ausgerichtet ist, ohne dass sich daraus die oben erläuterten Nachteile ergeben.

Die Aufgaben und Ziele werden hinsichtlich einer gattungsgemäßen medizinischen Fluidpumpe offenbarungsgemäß durch den Gegenstand des Anspruchs 1 gelöst.

Die medizinische Fluidpumpe, insbesondere in Form einer Infusions- oder Spritzenpumpe beinhaltet ein Gehäuse, eine an einer Gehäusevorderseite angelenkte Frontklappe, einen schwenkbar an sich gegenüberliegenden ersten und zweiten Seitenflächen anscharnierten Tragegriff, der aus einer Lagerungsposition in eine Trageposition schwenkbar ist, in welcher er sich oberhalb des Gehäuses anordnet und eine Anzahl von elektrischen und mechanischen Bauteilen innerhalb des Gehäuses, die für sämtliche vorgesehenen Funktionen der medizinischen Fluidpumpe erforderlich sind. Eine Auswahl an Bauteilen aus den elektrischen und mechanischen Bauteilen innerhalb des Gehäuses ist dabei als Tarierelemente vorgesehen, die innerhalb des Gehäuses so platziert sind, dass sich der Schwerpunkt der Fluidpumpe im Wesentlichen unterhalb des in Trageposition befindlichen Tragegriffs befindet.

In anderen Worten beinhaltet die medizinische Fluidpumpe, welche vorzugsweise als Spritzen- oder Schlauchpumpe ausgebildet ist, das Gehäuse mit einer an der Gehäusevorderseite mittels Scharnieren angelenkten Frontklappe, welche vorzugsweise ein Display in Form eines Touchdisplays aufweist. An den zwei Seitenflächen, die sich von äußeren Rändern der Gehäusevorderseite aus in Richtung einer Gehäuserückseite erstrecken, ist der Tragegriff, der vorzugsweise eine Bügelform aufweist, anscharniert. Der Tragegriff kann demnach von einer eingeklappten Position, bei der der Tragegriff an dem Gehäuse anliegt, in eine Trageposition geklappt werden, bei der der Tragegriff sich oberhalb des Gehäuses befindet. Innerhalb des Gehäuses befinden sich die elektrischen und mechanischen Bauteile der mechanischen Fluidpumpe, die zur Umsetzung aller insbesondere technischen Funktionen notwendig sind. Von diesen elektrischen und mechanischen Bauteilen wird eine Anzahl an Bauteilen ausgewählt und diese so in dem Gehäuse angeordnet, dass der Schwerpunkt sich im Wesentlichen in einer vertikalen Ebenen, in der auch der Tragegriff sich in der Trageposition befindet, liegt. Dies hat zur Folge, dass das Gehäuse sich an dem Tragegriff hängend im Wesentlichen in Waage befindet bzw. sich selbstständig im Wesentlichen horizontal ausrichtet.

Kern der Erfindung ist demnach, dass eine Auswahl der elektrischen und mechanischen Bauteile, die zu einer Umsetzung der an die medizinische Fluidpumpe gestellten Aufgaben notwendig sind, als Ausgleichs- bzw. Tariergewichte verwendet werden. Auf diese Weise kann gewährleistet werden, dass die medizinische Fluidpumpe bzw. das Gehäuse der medizinischen Fluidpumpe in eine gewünschte Position / Orientierung in Relation zu einer Umgebung gebracht wird, wenn sich diese hängend an dem Tragegriff befindet, ohne dass zusätzliche Bauteile wie beispielsweise (Blei-)Gewichte oder einrastende Scharniere oder dergleichen notwendig sind.

Bei den elektrischen und/oder mechanischen Bauteilen kann es sich sämtlich um für die Funktion der medizinischen Fluidpumpe erforderliche Bauteile handeln.

Dies ist auch deshalb wünschenswert, da mehrere der medizinischen Fluidpumpen untereinander gehängt werden können und dann an dem Tragegriff der obersten medizinischen Fluidpumpe getragen werden können. Wenn sich die Schwerpunkte der untereinander hängenden medizinischen Fluidpumpen im Wesentlichen in einer Ebenen mit dem sich in der Trageposition befindenden Tragegriff befinden, ist ein komfortables Tragen möglich. Wenn sich hingegen die Schwerpunkte der untereinander hängenden medizinischen Fluidpumpen nicht unterhalb des Tragegriffs befinden, führt dies zu einer unergonomischen Trageposition, welche ein schnelles Ermüden des Trägers zur Folge haben kann.

In einem ersten Aspekt kann die Auswahl an Bauteilen aus den elektrischen und mechanischen Bauteilen zumindest einen Energiespeicher und / oder ein Netzteil beinhalten.

In anderen Worten können zumindest der Energiespeicher und / oder das Netzteil als Tariergewichte genutzt werden. In nochmals anderen Worten kann durch die geeignete Platzierung von dem Energiespeicher und / oder dem Netzteil die Position des Schwerpunkts der medizinischen Fluidpumpe dahingehend beeinflusst werden, dass sich der Schwerpunkt im Wesentlichen unter dem sich in Trageposition befindlichen Tragegriff befindet. Da es sich bei dem Energiespeicher, welcher insbesondere als Akku beispielsweise in Form eines Bleiakkus, eines Nickel-Metallhydrid-Akkus, eines Lithium-Ionen-Akkus, eines Lithium-Polymer-Akkus oder eines Lithium-Metall.Akkus ausgebildet ist, und dem Netzteil um Bauteile handelt, die aufgrund der in ihnen verwendeten Werkstoffe ein hohes spezifisches Gewicht aufweisen, jedoch in ihrer Platzierung relativ flexibel innerhalb der medizinischen Fluidpumpe sind, sind gerade diese Bauteile besonders geeignet, um den Schwerpunkt der medizinischen Fluidpumpe gezielt zu manipulieren bzw. zu beeinflussen.

In einem weiteren Aspekt kann die Auswahl an Bauteilen aus den elektrischen und mechanischen Bauteilen zumindest einen Antriebsstrang und / oder einen Motor beinhalten.

In anderen Worten können zumindest der Antriebsstrang und / oder der Motor als Tariergewichte genutzt werden. In nochmals anderen Worten kann durch die geeignete Platzierung von dem Antriebsstrang und /oder dem Motor die Position des Schwerpunkts der medizinischen Fluidpumpe dahingehend beeinflusst werden, dass sich der Schwerpunkt im Wesentlichen unter dem sich in Trageposition befindlichen Tragegriff befindet. Da es sich bei dem Antriebsstrang, welcher insbesondere als linear zu dem Gehäuse bewegbarer Haltearm mit einem Antriebskopf oder als eine Peristaltik ausgebildet ist und bei dem den Antriebsstrang antreibenden Motor um Bauteile handelt, die aufgrund der in ihnen verwendeten Werkstoffe ein hohes spezifisches Gewicht und zusätzlich ein hohes absolutes Gewicht aufweisen, sind diese Bauteile geeignet, den Schwerpunkt der medizinischen Fluidpumpe gezielt zu manipulieren bzw. zu beeinflussen.

In einem weiteren Aspekt kann eine erste Baugruppe, vorzugsweise in Form des Antriebsstrangs, in einer der Vorderseite des Gehäuses zugewandten ersten Längshälfte des Gehäuses ausgebildet sein und eine zweite Baugruppe, vorzugsweise in Form des Netzteils und / oder eines Kommunikationsmoduls in einer der Vorderseite des Gehäuses gegenüberliegenden Hinterseite des Gehäuses zugewandten zweiten Längshälfte des Gehäuses ausgebildet sein, wobei ein Gewicht der ersten Baugruppe ein Gewicht der zweiten Baugruppe, bezogen auf eine Verkippungsachse durch die Anlenkpunkte, austariert.

In anderen Worten kann die erste Baugruppe, bei der es sich insbesondere um den Antriebsstrang handeln kann, in einem Inneren des Gehäuses in der ersten Längshälfte angeordnet sein, die direkt an die Frontklappe angrenzt. Die zweite Baugruppe, bei der es sich insbesondere um das Netzteil und / oder das Kommunikationsmodul handeln kann, in dem Inneren des Gehäuses in der zweiten Längshälfte angeordnet sein, welche zwischen der Hinterseite des Gehäuses und der ersten Längshälfte angeordnet ist. Ein erstes Moment, das durch eine erste Gewichtskraft der ersten Baugruppe bezogen auf die Verkippungsachse durch die Anlenkpunkte des Tragegriffs erzeugt wird, wird im Wesentlichen durch ein zweites Moment, das durch eine zweite Gewichtskraft der zweiten Baugruppe bezogen auf die Verkippungsachse durch die Anlenkpunkte des Tragegriffs erzeugt wird, aufgehoben. In nochmals anderen Worten herrscht im Wesentlichen ein Momentengleichgewicht der ersten Gewichtskraft und der zweiten Gewichtskraft, bezogen auf die Verkippungsachse.

In einem weiteren Aspekt kann eine dritte Baugruppe, welche vorzugsweise in Form des Antriebskopfes außerhalb des Gehäuses an der zweiten Seitenfläche ausgebildet sein kann, sich relativ zu dem Gehäuse mittels eines Antriebsarmes linear zu bewegen und eine vierte Baugruppe, vorzugsweise in Form des Motors, in einer von dem Antriebskopf abgewandten ersten Querhälfte des Gehäuses angeordnet sein.

In anderen Worten kann die dritte Baugruppe, bei welcher es sich vorzugsweise um den Antriebskopf handelt, welcher mittels des Haltearms linear zu dem Gehäuse bewegbar ist, außerhalb des Gehäuses der medizinischen Fluidpumpe angrenzend an die zweite Seitenfläche ausgebildet sein. Die vierte Baugruppe, welche insbesondere als der Motor ausgebildet sein kann, der für ein Antreiben des Antriebskopfes verantwortlich ist, kann in der ersten Querhälfte des Innenraums des Gehäuses angeordnet sein, welche an die erste Seitenfläche angrenzt und von der zweiten Seitenfläche distanziert angeordnet ist.

In nochmals anderen Worten kann der Innenraum des Gehäuses in eine erste Querhälfte und eine zweite Querhälfte unterteilt werden. Die erste Querhälfte grenzt an die erste Seitenfläche und an die zweite Querhälfte an und beinhaltet die vierte Baugruppe, bei welcher es sich insbesondere um den Motor handelt. Die zweite Querhälfte grenzt an die zweite Seitenfläche und die erste Querhälfte an. Außerhalb des Gehäuses, angrenzend an die zweite Seitenfläche, welche die zweite Querhälfte begrenzt, kann eine dritte Baugruppe, vorzugsweise in Form des Antriebskopfes, angeordnet sein. Auf diese Weise sind die dritte Baugruppe und die vierte Baugruppe möglichst weit voneinander entfernt angeordnet. Einerseits kann dadurch die vierte Baugruppe als eine Art Gegengewicht für die dritte Baugruppe fungieren, insbesondere, wenn die dritte Baugruppe ausgefahren ist. Andererseits kann so effektiv verhindert werden, dass Schwingungen, welche in der vierten Baugruppe entstehen, an die dritte Baugruppe weitergeleitet werden und diese in ihrer Funktion beeinträchtigen.

In einem weiteren Aspekt kann der Energiespeicher im Wesentlichen in dem Schwerpunkt der medizinischen Fluidpumpe angeordnet sein.

In anderen Worten ist der Energiespeicher im Wesentlichen unter dem sich in Trageposition befindlichen Tragegriff angeordnet. Da es sich bei dem Energiespeicher um ein Bauteil mit einem hohen spezifischen und absoluten Gewicht handelt, ist es vorteilhaft, diesen in dem gewünschten Schwerpunkt der medizinischen Fluidpumpe anzuordnen.

In einem weiteren Aspekt kann das Kommunikationsmodul mit einer ersten Antenne und einer zweiten Antenne ausgebildet / verbunden sein, wobei die erste Antenne an der ersten Seitenfläche ausgebildet ist und die zweite Antenne an der zweiten Seitenfläche ausgebildet ist.

In anderen Worten kann die medizinische Fluidpumpe das Kommunikationsmodul beinhalten, welches über zwei externe Antennen verfügen kann. Die erste Antenne ist an der ersten Seitenfläche vorzugsweise in einer ersten Antennenaufnahme fixiert und die zweite Antenne ist an der zweiten Seitenfläche in einer zweiten Antennenaufnahme fixiert. Durch eine solche Anordnung der Antennen können mögliche Beeinflussungen der Übertragungsleistung der Antennen aufeinander verhindert werden.

In einem weiteren Aspekt kann der Abstand zwischen der ersten Seitenfläche und der zweiten Seitenfläche auf die Übertragungsfrequenz der ersten und der zweiten Antenne abgestimmt sein. Vorzugsweise entspricht der Abstand im Wesentlichen einem Vielfachen einer viertel, insbesondere vorzugsweise einem Vielfachen einer halben Wellenlänge der Übertragungsfrequenz.

In anderen Worten beträgt die Übertragungsfrequenz der Antennen 2,4 GHz und / oder 5 GHz. Die Wellenlänge beträgt für 2,4 GHz 12,5 cm und für 5 GHz 6 cm. Der Abstand zwischen den Antennen und damit zwischen der ersten Seitenfläche und der zweiten Seitenfläche beträgt demnach ein Vielfaches von 3,125 cm bzw. ein Vielfaches von 3 cm.

In nochmals anderen Worten sind die Abmessungen, insbesondere eine Breitenabmessung der medizinischen Fluidpumpe durch die Übertragungsfrequenz der Antennen des Kommunikationsmoduls vorgegeben. Aus diesem Grund lassen sich die Abmessungen der medizinischen Fluidpumpe nicht beliebig verändern, sondern sind im Wesentlichen vorgegeben.

In einem weiteren Aspekt kann die vierte Baugruppe, vorzugsweise in Form des Motors, in der ersten Querhälfte des Gehäuses angeordnet sein und eine fünfte Baugruppe, vorzugsweise in Form eines Schließriegelantriebs, in der zweiten, von der ersten Querhälfte verschiedenen Querhälfte des Gehäuses angeordnet sein.

In anderen Worten sind die vierte Baugruppe, vorzugsweise in Form des Motors, und die fünfte Baugruppe, vorzugsweise in Form des Schließriegelantriebs, in unterschiedlichen Querhälften des Innenraums des Gehäuses der medizinischen Fluidpumpe angeordnet. Auf diese Weise sind die vierte Baugruppe und die fünfte Baugruppe möglichst weit voneinander entfernt angeordnet. Auf diese Weise kann effektiv verhindert werden, dass Schwingungen, welche in der vierten Baugruppe oder in der fünften Baugruppe entstehen, an die jeweils andere der vierten und fünften Baugruppe weitergeleitet werden und diese in ihrer Funktion beeinträchtigen.

### Kurzbeschreibung der Figuren

Fig. 1 ist eine perspektivische Ansicht einer offenbarungsgemäßen medizinischen Fluidpumpe, hier beispielhaft als Infusionspumpe bzw. Schlauchpumpe dargestellt.
Fig. 2 ist eine Seitenansicht einer offenbarungsgemäßen medizinischen Fluidpumpe, hier beispielhaft als Infusionspumpe bzw. Schlauchpumpe dargestellt.
Fig. 3 ist eine perspektivische Ansicht von drei offenbarungsgemäßen medizinischen Fluidpumpen, die in einem Verbund von einem Bediener getragen werden.
Fig. 4 ist eine Darstellung eines Innenraums einer medizinischen Fluidpumpe in Form einer Spritzenpumpe.
Fig. 5 ist eine Darstellung eines Innenraums einer medizinischen Fluidpumpe, in Form einer Infusionspumpe bzw. Schlauchpumpe.

Die Figuren sind schematischer Natur und dienen lediglich dem Verständnis der Offenbarung. Gleiche Elemente sind mit denselben Bezugszeichen versehen.

### Detaillierte Beschreibung anhand der Figuren

Nachfolgend werden bevorzugte Ausführungsformen anhand der Figuren beschrieben. Hierbei werden Merkmale und Eigenschaften beispielhaft an einzelnen Ausführungsformen beschrieben. Es sei explizit erwähnt, dass hier beschriebene Merkmale nicht auf die jeweilige Ausführungsform beschränkt sind.

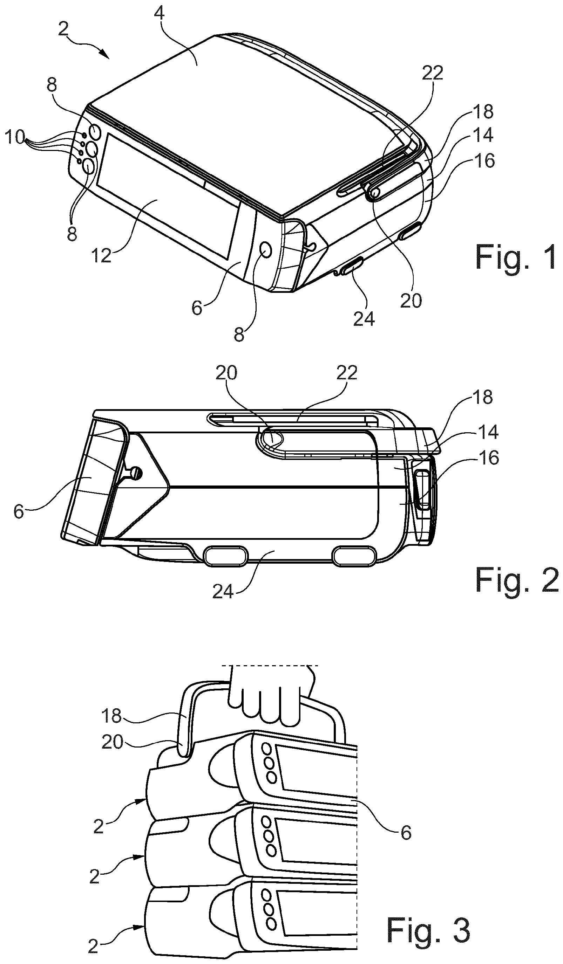

Fig. 1 zeigt eine medizinische Fluidpumpe 2 gemäß einer bevorzugten Ausführungsform. Dabei ist die in Fig. 1 dargestellte medizinische Fluidpumpe 2 als Infusionspumpe ausgeführt. Selbstverständlich kann die medizinische Fluidpumpe auch als Spritzenpumpe ausgebildet sein. Wie in Fig. 1 gezeigt, weist die medizinische Fluidpumpe 2 ein im Wesentlichen quaderförmiges Gehäuse 4 auf, an dessen Vorderseite eine an dem Gehäuse 4 schwenkbar angelenkte Frontklappe 6 ist. Auf einer Vorderseite der Frontklappe 6 sind mehrere Bedientasten 8, mehrere Signalleuchten 10 und ein Touchdisplay 12 angeordnet.

Das Gehäuse 4 weist ferner eine Gehäuseoberschale 14 und ein Gehäuseunterteil 16 auf, welche miteinander verbunden werden können und so das Gehäuse 4 ausbilden. An der Gehäuseoberschale 14 ist ein als Griffbügel ausgeführter Tragegriff 18 schwenkbar angelenkt. Dabei umgreift der Tragegriff 18 die Gehäuseoberschale 14 in einer Breitenrichtung, sodass der Tragegriff 18 an beiden Seitenflächen bzw. an einer ersten Seitenfläche und einer zweiten Seitenfläche der Gehäuseoberschale 14 bzw. des Gehäuses 4 angelenkt ist. Der Tragegriff 18 ist hierbei in Anlenkpunkten 20 mit dem Gehäuse 4 verbunden. Die Anlenkpunkte 20 sind als Scharniere ausgeführt und befinden sich, wie in Fig. 1 und Fig. 2 dargestellt, im Wesentlichen mittig in einer Pumpenlängsrichtung. Als Pumpenlängsrichtung ist eine Richtung ausgehend von der Vorderseite mit der Frontklappe 6 hin zu einer der Vorderseite gegenüberliegenden Rückseite der medizinischen Fluidpumpe 2 zu verstehen. Bei einem Ausklappen des Tragegriffs 18 aus der Lagerposition in eine Trageposition verschwenkt der Tragegriff um die Anlenkpunkte 20. Die Drehachse geht damit durch die Anlenkpunkte 20.

Fig. 2 ist eine Darstellung einer Seitenansicht der medizinischen Fluidpumpe 2. In der Gehäuseoberschale sind obere Rastschienen 22 in der ersten Seitenfläche und der zweiten Seitenfläche ausgebildet. In der Gehäuseunterschale sind untere Rastschienen 24 in der ersten Seitenfläche und der zweiten Seitenfläche ausgebildet. Die unteren Rastschienen 24 einer ersten medizinischen Fluidpumpe 2 sind dafür vorgesehen und ausgebildet, in die oberen Rastschienen einer zweiten, baugleichen medizinischen Fluidpumpe 2 einzugreifen und die erste medizinische Fluidpumpe 2 und die zweiten medizinische Fluidpumpe 2 aneinander zu koppeln.

In Fig. 1 und Fig. 2 ist die medizinische Fluidpumpe in einem Zustand dargestellt, in dem der Tragegriff 18 eingeklappt ist und an der Gehäuseoberschale 14 anliegt. Der Tragegriff 18 schließt in dem eingeklappten Zustand eben mit den Seitenflächen ab.

Wie in Fig. 3 gezeigt, können gemäß dem bevorzugten Ausführungsbeispiel drei medizinische Fluidpumpen 2 übereinandergestapelt, miteinander verbunden/gekoppelt und mit dem Tragegriff 18 der obersten medizinischen Fluidpumpe 2 getragen werden. Das heißt der Tragegriff 18 der medizinischen Fluidpumpe 2 ist ausgelegt und dimensioniert, um einen Verbund aus drei miteinander gekoppelten medizinischen Fluidpumpen 2 zu tragen. Der Tragegriff 18 der in Fig. 3 dargestellten obersten medizinischen Fluidpumpe 2 befindet sich in der Trageposition. Erfindungsgemäß ist ein jeweiliger Schwerpunkt der medizinischen Fluidpumpen in einer im Wesentlichen vertikal angeordneten Ebenen mit dem Tragegriff 18. Das Gehäuse 4 ist in der Trageposition im Wesentlichen horizontal ausgerichtet. Der Tragegriff 18 ist in der Trageposition im Wesentlichen vertikal ausgerichtet.

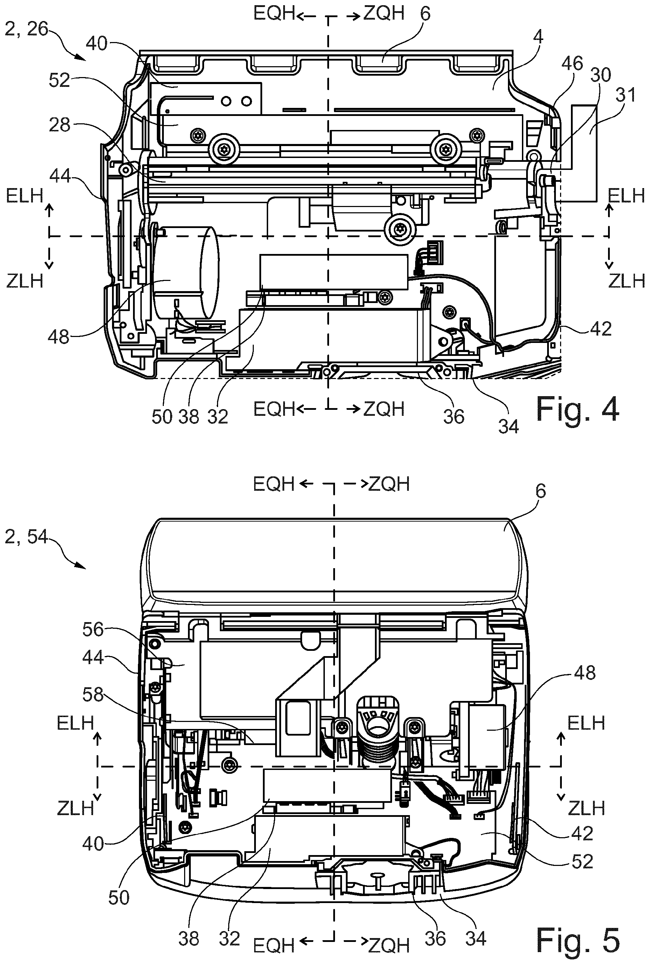

Fig. 4 zeigt den inneren Aufbau der offenbarungsgemäßen medizinischen Fluidpumpe 2 in Form einer Spritzenpumpe 26. Die Spritzenpumpe 26 beinhaltet einen Antriebsstrang 28, der vorgesehen und ausgebildet ist, einen Haltearm 30, an dessen Ende ein Antriebskopf 31 vorgesehen ist, der linear zu dem Gehäuse 4 verfahren werden kann. Der Antriebsstrang 28 ist in einer der Gehäusevorderseite und damit der Frontklappe 6 zugewandten ersten Längshälfte ELH des Gehäuses 4 ausgebildet. Ein Netzteil 32 ist in einer zweiten Längshälfte ZLH des Gehäuses 4 ausgebildet. Die zweite Längshälfte ist einer der Frontklappe 6 gegenüberliegenden Hinterseite 34 zugewandt. Das Netzteil 32 ist in vorteilhafter Weise an der Hinterseite 34 anliegend ausgebildet. Dies ermöglicht ein Vorsehen von Kühlrippen 36 an der Hinterseite 34 des Gehäuses 4. Diese Kühlrippen 36 sind vorgesehen und ausgebildet, das Netzteil 32 zu Kühlen. Neben dem Netzteil 32 ist auch ein drahtloses Kommunikationsmodul 38 in der zweiten Längshälfte ZLH vorgesehen und ausgebildet. Das drahtlose Kommunikationsmodul 38 ist insbesondere zwischen dem Netzteil 32 und dem Antriebsstrang 28 angeordnet. In anderen Worten ist das drahtlose Kommunikationsmodul 38 zwischen der Hinterseite 34 und der ersten Längshälfte ELH angeordnet. Das drahtlose Kommunikationsmodul 38 ist mit einer ersten Antenne 40 und mit einer zweiten Antenne 42 verbunden, wobei die erste Antenne 40 angrenzend an eine erste Seitenfläche 44 des Gehäuses 4 vorgesehen und ausgebildet ist. Die zweite Antenne 42 ist anliegend an eine zweite Seitenfläche 46 des Gehäuses 4 vorgesehen und ausgebildet. Die erste Antenne 40 und die zweite Antenne 42 sind orthogonal zueinander orientiert. Der Abstand zwischen der ersten Seitenfläche 44 und der zweiten Seitenfläche 46 entspricht einem Vielfachen einer viertel bzw. einem Vielfachen einer halben Wellenlänge der Übertragungsfrequenz der ersten Antenne 40 und der zweiten Antenne 42. Ein Motor 48, der den Antriebsstrang 28 antreibt, ist in einer ersten Querhälfte EQH des Gehäuses 4 ausgebildet. Der Antriebskopf 31 grenzt an die zweite Querhälfte ZQH des Gehäuses 4 an. Ein Energiespeicher 50 ist zwischen dem drahtlosen Kommunikationsmodul 38 und dem Antriebsstrang 28 ausgebildet. Eine Hauptplatine 52 ist flächig in dem Gehäuse 4 angeordnet. Die Hauptplatine 52 beinhaltet wesentliche Steuerungsvorrichtungen für die Spritzenpumpe 26.

Fig. 5 zeigt einen inneren Aufbau der medizinischen Fluidpumpe 2 in Form einer weiteren bevorzugten Ausführungsform. Genauer gesagt zeigt die Fig. 5 den inneren Aufbau der offenbarungsgemäßen medizinischen Fluidpumpe 2 vom Typ Infusionspumpe 54 bzw. Schlauchpumpe 54. Die Infusionspumpe 54 beinhaltet einen Antriebsstrang 56 in Form einer Peristaltik, welcher vorgesehen und ausgebildet ist, eine medizinische Flüssigkeit mittels einer Peristaltik zu fördern. Der Antriebsstrang 56 ist in der Gehäusevorderseite und damit der Frontklappe 6 zugewandten ersten Längshälfte ELH des Gehäuses 4 ausgebildet. Das Netzteil 32 ist in der zweiten Längshälfte ZLH des Gehäuses 4 ausgebildet. Die zweite Längshälfte ZLH ist der Frontklappe 6 gegenüberliegenden Hinterseite 34 zugewandt. Das Netzteil 32 ist in vorteilhafter Weise an der Hinterseite 34 anliegend ausgebildet. Dies ermöglicht ein Vorsehen der Kühlrippen 36 an der Hinterseite 34 des Gehäuses 4. Diese Kühlrippen 36 sind vorgesehen und ausgebildet, das Netzteil 32 zu kühlen. Neben dem Netzteil 32 ist auch ein drahtloses Kommunikationsmodul 38 in der zweiten Längshälfte ZLH vorgesehen und ausgebildet. Das drahtlose Kommunikationsmodul 38 ist insbesondere zwischen dem Netzteil 32 und der ersten Längshälfte ELH angeordnet. Das drahtlose Kommunikationsmodul 38 ist mit der ersten Antenne 40 und mit der zweiten Antenne 42 verbunden, wobei die erste Antenne 40 anliegend an die erste Seitenfläche 44 des Gehäuses 4 vorgesehen und ausgebildet ist. Die zweite Antenne 42 ist anliegend an die zweite Seitenfläche 46 des Gehäuses 4 vorgesehen und ausgebildet. Die erste Antenne 40 und die zweite Antenne 42 sind orthogonal zueinander orientiert. Der Abstand zwischen der ersten Seitenfläche 44 und der zweiten Seitenfläche 46 entspricht einem Vielfachen einer viertel bzw. einem Vielfachen einer halben Wellenlänge der Übertragungsfrequenz der ersten Antenne 40 und der zweiten Antenne 42. Der Motor 48, der den Antriebsstrang 56 antreibt, ist in der zweiten Querhälfte ZQH des Gehäuses 4 ausgebildet. Ein Schließriegelantrieb 58, ist in der ersten Querhälfte EQH des Gehäuses 4 ausgebildet. Ein Energiespeicher 50 ist zwischen dem drahtlosen Kommunikationsmodul 38 und dem Antriebsstrang 56 ausgebildet. Die Hauptplatine 52 ist flächig in dem Gehäuse 4 angeordnet. Die Hauptplatine 52 beinhaltet wesentliche Steuerungsvorrichtungen für die Infusionspumpe 54.

### Bezugszeichenliste

- 2: medizinische Fluidpumpe
- 4: Gehäuse
- 6: Frontklappe
- 8: Bedientaste
- 10: Signalleuchte
- 12: Touchdisplay
- 14: Gehäuseoberschale
- 16: Gehäuseunterteil
- 18: Tragegriff
- 20: Anlenkpunkt
- 22: obere Rastschiene
- 24: untere Rastschiene
- 26: Spritzenpumpe
- 28: Antriebsstrang
- 30: Antriebsarm / Haltearm
- 31: Antriebskopf
- 32: Netzteil
- 34: Hinterseite
- 36: Kühlrippe
- 38: drahtloses Kommunikationsmodul
- 40: erste Antenne
- 42: zweite Antenne
- 44: erste Seitenfläche
- 46: zweite Seitenfläche
- 48: Motor
- 50: Energiespeicher
- 52: Hauptplatine
- 54: Infusionspumpe / Schlauchpumpe
- 56: Antriebsstrang
- 58: Schließriegelantrieb
- ELH: erste Längshälfte
- ZLH: zweite Längshälfte
- EQH: erste Querhälfte
- ZQH: zweite Querhälfte

## Patentansprüche

1. Medizinische Fluidpumpe (2), insbesondere in Form einer Spritzen- oder Infusionspumpe (26; 54), mit
- einem Gehäuse (4),
- einem schwenkbar an dem Gehäuse (4) anscharnierten Tragegriff (18), der aus einer Lagerungsposition in eine Trageposition schwenkbar ist, in welcher er sich oberhalb des Gehäuses (4) anordnet und
- einer Anzahl von elektrischen und mechanischen Bauteilen innerhalb des Gehäuses (4), die vorgesehene Funktionen der medizinischen Fluidpumpe (2) ausführen,
**dadurch gekennzeichnet, dass**
eine Auswahl an Bauteilen aus den elektrischen und mechanischen Bauteilen innerhalb des Gehäuses (4), als Tarierelemente vorgesehen sind, die innerhalb des Gehäuses (4) so platziert sind, dass sich ein Schwerpunkt der medizinischen Fluidpumpe (2) im Wesentlichen unterhalb des in Trageposition befindlichen Tragegriffs (18) befindet.

2. Medizinische Fluidpumpe (2) nach Anspruch 1, **dadurch gekennzeichnet, dass** die Auswahl an Bauteilen aus den elektrischen und mechanischen Bauteilen zumindest einen Energiespeicher (50) und / oder ein Netzteil (32) beinhaltet.

3. Medizinische Fluidpumpe (2) nach Anspruch 1 oder 2, **dadurch gekennzeichnet, dass** die Auswahl an Bauteilen aus den elektrischen und mechanischen Bauteilen zumindest einen Antriebsstrang (28) und / oder einen Motor (48) enthält.

4. Medizinische Fluidpumpe (2) nach einem der Ansprüche 1 bis 3, **dadurch gekennzeichnet, dass** eine erste Baugruppe, vorzugsweise in Form des Antriebsstrangs (28), in einer der Vorderseite des Gehäuses (4) zugewandten ersten Längshälfte (ELH) des Gehäuses (4) ausgebildet ist und eine zweite Baugruppe, vorzugsweise in Form des Netzteils (32) und / oder eines Kommunikationsmoduls (38) in einer der Vorderseite des Gehäuses (4) gegenüberliegenden Hinterseite des Gehäuses (4) zugewandten zweiten Längshälfte (ZLH) des Gehäuses (4) ausgebildet ist, wobei ein Gewicht der ersten Baugruppe ein Gewicht der zweiten Baugruppe, bezogen auf eine Rotationsachse durch die Anlenkpunkte (20), austariert.

5. Medizinische Fluidpumpe (2) nach einem der Ansprüche 1 bis 4, **dadurch gekennzeichnet, dass** eine dritte Baugruppe, welche vorzugsweise in Form eines Antriebskopfes (31) außerhalb des Gehäuses (4) an der zweiten Seitenfläche (46) ausgebildet ist, sich relativ zu dem Gehäuse (4) mittels eines Antriebsarmes (30) linear zu bewegen und eine vierte Baugruppe, vorzugsweise in Form des Motors (48), in einer von dem Antriebskopf (31) abgewandten ersten Querhälfte (EQH) des Gehäuses (4) angeordnet ist.

6. Medizinische Fluidpumpe (2) nach einem der Ansprüche 1 bis 5, **dadurch gekennzeichnet, dass** der Energiespeicher (50) im Wesentlichen in dem Schwerpunkt der medizinischen Fluidpumpe (2) angeordnet ist.

7. Medizinische Fluidpumpe (2) nach Anspruch 4, **dadurch gekennzeichnet, dass** das Kommunikationsmodul (38) mit einer ersten Antenne (40) und einer zweiten Antenne (42) verbunden / ausgebildet ist, wobei die erste Antenne (40) an der ersten Seitenfläche (44) ausgebildet ist und die zweite Antenne (42) an der zweiten Seitenfläche (46) ausgebildet ist.

8. Medizinische Fluidpumpe (2) nach Anspruch 7, **dadurch gekennzeichnet, dass** ein Abstand zwischen der ersten Seitenfläche (44) und der zweiten Seitenfläche (46) auf die Übertragungsfrequenz der ersten und zweiten Antenne (40; 42) abgestimmt ist und vorzugsweise im Wesentlichen einem Vielfachen einer viertel, insbesondere vorzugsweise einem Vielfachen einer halben Wellenlänge der Übertragungsfrequenz entspricht.

9. Medizinische Fluidpumpe (2) nach Anspruch 1 oder 2, **dadurch gekennzeichnet, dass** die vierte Baugruppe, vorzugsweisen in Form des Motors (48), in einer zweiten, von der ersten Querhälfte (EQH) verschiedenen Querhälfte (ZQH) des Gehäuses (4) angeordnet ist und eine fünfte Baugruppe, vorzugsweise in Form eines Schließriegelantriebs (58) in der ersten Querhälfte (EQH) des Gehäuses (4) angeordnet ist.
